# EUROPEAN PATENT APPLICATION

(11) **EP 0 904 765 A2**
(43) Date of publication of application: **31.03.1999**
(21) Application number: 98250329.4
(22) Date of filing: 15.09.1998
(51) Int. Cl.: A61K 6/04

(54) **Gold coloured dental alloy**

(30) Priority: 25.09.1997 US 936400
(71) Applicant: IVOCLAR AG, 9494 Schaan (LI)
(72) Inventor: Schaffer, Stephen P., Hamburg, NY 14075 (US); McCabe, Patrick J., North Tonawanda, NY 14120 (US)
(74) Representative: UEXKÜLL & STOLBERG

(57) **Abstract**

A palladium-free, yellow dental alloy is disclosed with high gold content for castings and resin veneers. The dental alloy exhibits high mechanical strength and hardness while achieving maximum yellow color and bonding with the resin. The alloy contains 60 to 90% by weight gold, 0 to 20% by weight silver, 0 to 7% by weight palladium, 0 to 11% by weight platinum, 0 to 20% by weight copper, 0 to 2% by weight zinc, 0 to 1% by weight tin, 0 to 3% by weight indium and trace amounts of a grain refiner.

## Description

### Background of the invention

The present invention relates to a yellow dental alloy with a high gold content for prosthetic parts with or without a veneer. Alloys of precious metals are widely used for making metallic, fixed dentures and dental prostheses due to their favourable properties such as excellent corrosion resistance, bio-compatibility and good workability and processibility. Cast gold alloys have been used for many decades in dentistry. Cast alloys with a high gold content contain additional elements besides gold in order to obtain varying degrees of hardness and strength. ISO 1562 categorizes cast gold alloys into Type I, Type II, Type III, and Type IV based on degrees of strength and elongation. Type I describes the alloys with the least strength which are useful for low stress applications such as inlays and small onlays. Type IV alloys have the greatest strength and are used in many more applications including those subjected to the highest stresses such as long span bridges and partial dentures. Alloys with Type IV properties have a much broader range of indications due to their higher mechanical stability.

Conventional cast alloys with Type IV properties derive their strength from the hardenability of the gold-silver-copper (Au-Ag-Cu) ordering system coupled with additions of platinum and palladium. In addition to strengthening the alloys, the platinum and palladium also increase the corrosions resistance of the alloys. Palladium has been shown to be more effective, at lower concentrations, in increasing the strength of Au-Ag-Cu alloys than has platinum. However, additions of more than 5% by weight of palladium or 10 % platinum cause the alloy to lose its warm, very yellow color and the resultant alloys are either only of a light yellow color or are white in color. Additionally, there has recently been concern among dental practitioners that there may be a portion of the population which may have severe allergic reactions to palladium. Although palladium is typically a biocompatible material, those with allergic sensitivities to palladium can exhibit a vast array of physical maladies when exposed to palladium.

Moreover, for esthetic reasons, it has become popular to cover portions of the alloys with a resin veneer so that it resembles the color texture and translucency of natural tooth structure. Traditionally, these resins are held to the surface of the alloy with mechanical retention such as beads or loops which are cast as part of the alloy surface. The disadvantage of this situation is that there is no chemical bond of the resin to the alloy. Oral fluids and debris are able to flow between the veneer and the alloy and cause discoloration and occasionally fracture of the veneer.

It would, therefore, be of great advantage to have a dental alloy displaying the advantages of the cast alloy with regard to color, strength and processing and, moreover, which could be bonded with the resin veneering material.

### Summary of the invention

The primary object of the present incention is to provide a high gold content dental casting alloy for fabricating prosthetic parts, crowns, bridges and/or implants, with or without a veneer. Another object of the present invention, is to provide such an alloy having a deep yellow color. Another object of the present invention, is to provide such an alloy possessing Type IV properties. Another object of the present invention, is to provide such an alloy which is capable of bonding with the veneering material thereby maintaining the high esthetic appearance of the restoration.

Acording to the present invention, this and other objects are achieved by providing an alloy comprising 60 to 90% by weight gold, 0 to 20% by weight silver, 0 to 7% by weight palladium, 0 to 11% by weight platinum, 0 to 20% by weight copper, 0 to 2% by weight zinc, 0 to 1% by weight tin and 0 to 3% by weight indium. Optionally, the alloys of the invention comprises trace amounts of a grain refiner.

### Detailed description of the invention

The invention provides a yellow dental alloy having a high gold content comprising 60 to 90 % by weight gold, 0 to 20 % by weight silver, 0 to 7% by weight palladium, 0 to 11% by weight platinum, 0 to 20% by weight copper, 0 to 2% by weight zinc, 0 to 1% by weight tin and 0 to 3% by weight indium. Optionally, the dental alloy of the invention comprises trace amounts of a grain refiner comprising 0.001 to 0.1% by weight of the formulation. More preferably the grain refiner comprises 0.001 to 0.1% by weight iridium and most preferably, 0.01% by weight iridium.

More preferably, the alloy of the invention comprises 60 to 72% by weight gold, 10 to 17% by weight silver, 0 to 4% by weight platinum, 9 to 17% by weight copper, 0.9 to 1.1% by weight zinc, 0.1 to 1% by weight tin, 0 to 1% by weight indium and 0.001 to 0.1% by weight a grain refiner.

Most preferably, the alloy of the invention comprises 70.7% by weight gold, 13.7% by weight silver, 3.59% by weight platinum, 10% by weight copper, 1% by weight zinc, 1% by weight tin and 0.01% by weight iridium.

By balancing the ratio of silver to copper and increasing the platinum content the desirable result of Type IV properties with a very warm, deep yellow color can be achieved. By the addition of small amounts of tin and zinc the bonding of the resin can be maximized without sacrificing either the strength or the color of the alloy.
The following table shows the formulations of some alloys according to the invention.

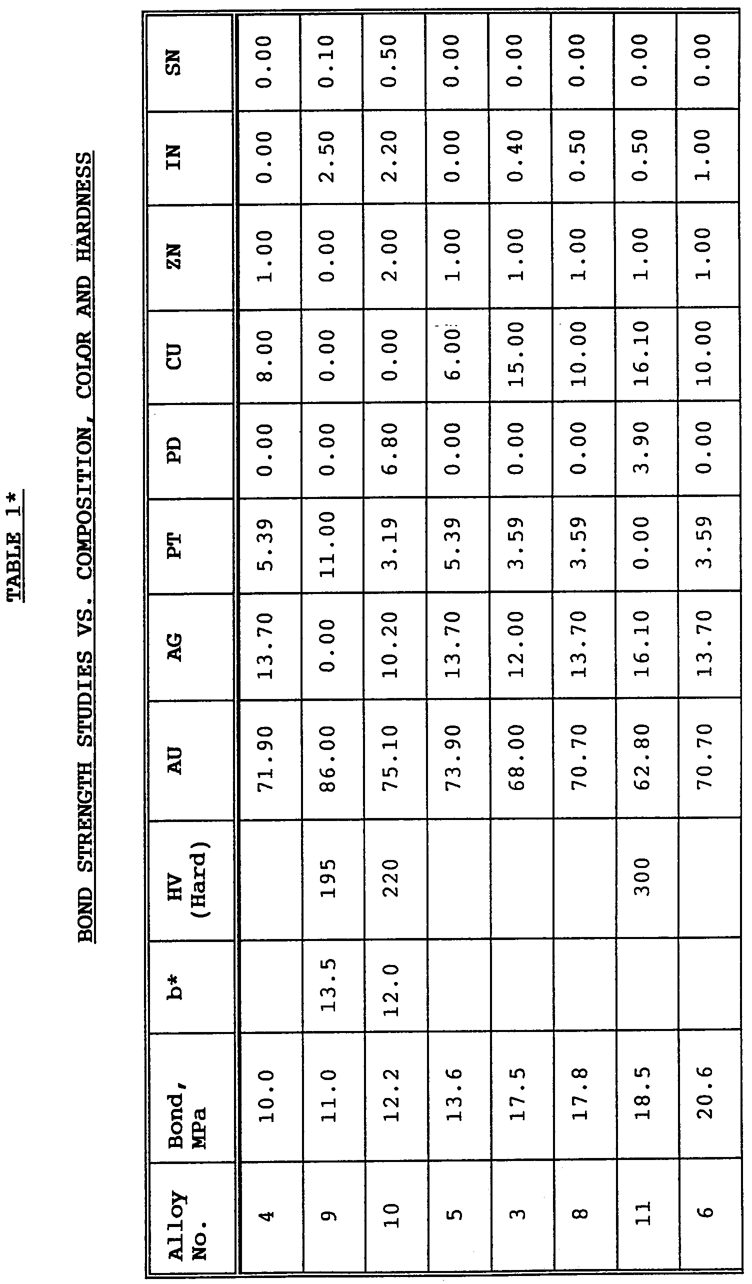

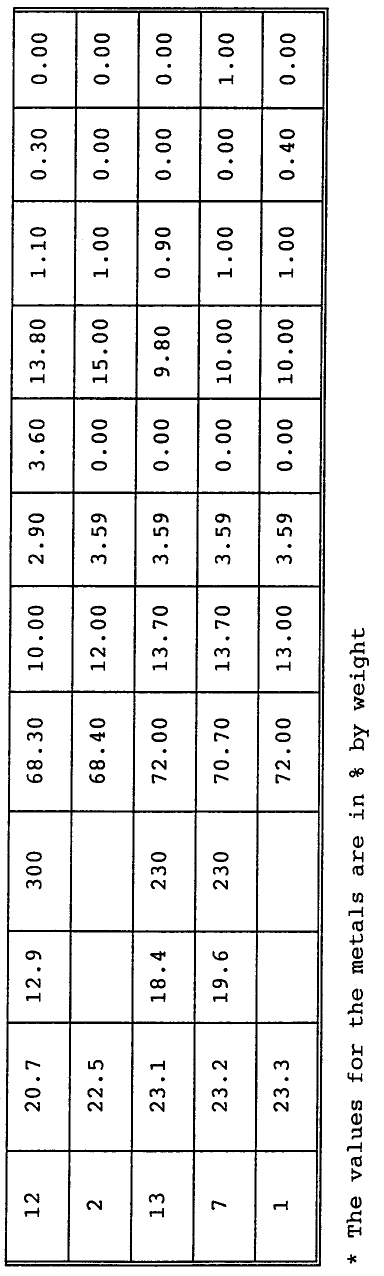

The following table shows preferred physical physical properties of some alloys of the invention.

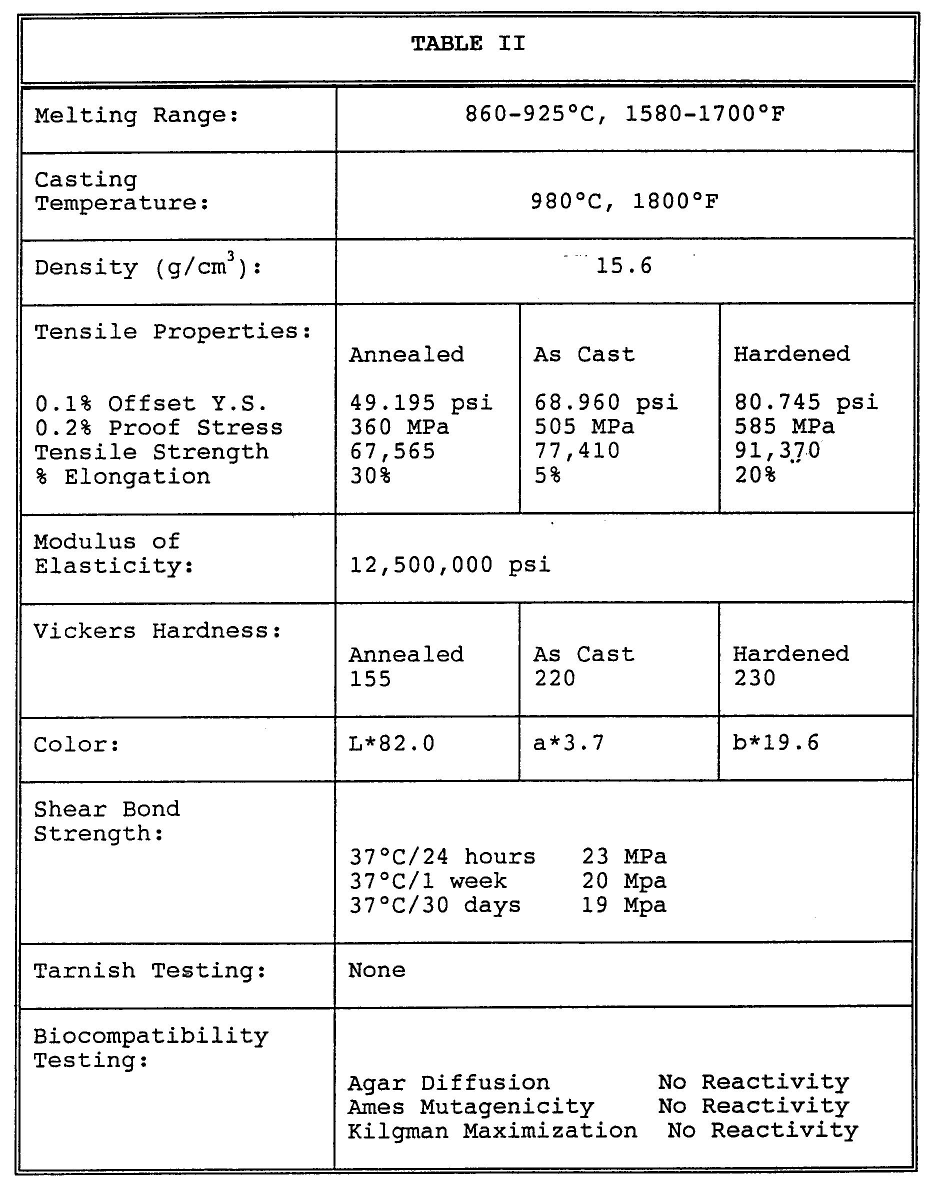

The alloys of the invention exhibit the following advantages and/or benefits over conventional cast alloys: (1) High yield strength, elongation and Vickers hardness; (2) Provide strength suitable for milling telescope crowns and creating implant superstructures; (3) Can be locally oxidized through sandblasting; (4) Creates a superior bond to resin veneering materials; (5) High noble alloy; and (6) Good polishing characteristics. The alloys of the invention are preferably Type IV having indications including, without limitation, restorations subject to very high stress, such as bridges, inlays, onlays, crowns, thick veneer crowns and short span fixed partial dentures.

The present invention also concerns a method of making dental prostheses using the dental alloy described above. Known casting methods in the art are employed to form the dental prostheses. Any known ceramic (porcelain) and/or resin material which is compatible with the physical properties of the present alloys can be used to fabricate the dental prostheses, dental bridge, crown or other dental article. Preferably, a ceramic optimized polymer material (Ivoclar North America, Inc., Amherst, N.Y.) is used.

The present invention further concerns the dental prostheses, dental bridge, dental crown or other dental items formed from the alloy described above by methods known in the art.

Further variations and modifications of the invention will become apparent to those skilled in the art from the foregoing and are intended to be encompassed by the claims appended hereto.

## Claims

1. A yellow dental alloy with a high gold content for prosthetic parts with or without a veneer, consisting essentially of 60 to 90% by weight of gold, 0 to 20% by weight silver, 0 to 7% by weight palladium, 0 to 11% by weight platinum, 0 to 20% by weight copper, 0 to 2% by weight zinc, 0 to 1% by weight tin and 0 to 3% by weight indium and trace amounts of a grain refiner.

2. The dental alloy according to claim 1, comprising 60 to 72% by weight gold, 10 to 17% by weight silver, 3 to 7% by weight palladium, 0 to 4% by weight platinum, 9 to 17% by weight copper, 0.9 to 1.1% by weight zinc, 0.1 to 1% by weight tin, 0 to 1% by weight indium and 0.001 to 0.1% by weight grain refiners.

3. The dental alloy according to claim 1, comprising 70.7% weight gold, 13.7% by weight silver, 3.59% by weight platinum, 10% by weight copper, 1% by weight zinc, 1% by weight tin and 0.01% by weight iridium.

4. A dental prostheses formed of the dental alloy according to any one of claims 1 to 3.

5. A dental bridge formed of the dental alloy according to any one of claims 1 to 3.

6. A dental crown formed of the dental alloy according to any one of claims 1 to 3.

7. A method of making dental prostheses comprising casting a dental alloy consisting essentially of 60 to 90% by weight gold, 0 to 20% by weight silver, 0 to 7% by weight palladium, 0 to 11% by weight platinum, 0 to 20% by weight copper, 0 to 2% by weight zinc, 0 to 1% by weight tin, 0 to 3% by weight indium and trace amounts of a grain refiner to form said dental prostheses.

8. A dental prostheses formed of a dental alloy consisting essentially of 60 to 90% by weight gold, 0 to 20% by weight silver, 0 to 7% by weight palladium, 0 to 11% by weight platinum, 0 to 20% by weight copper, 0 to 2% by weight zinc, 0 to 1% by weight tin, 0 to 3% by weight indium and trace amounts of a grain refiner in combination with a veneer.

9. The dental prosthesis of claim 8, wherein said alloy is bonded to said veneer.

10. A dental bridge formed of a dental alloy consisting essentially of 60 to 90% by weight gold, 0 to 20% by weight silver, 0 to 7% by weight palladium, 0 to 11% by weight platinum, 0 to 20% by weight copper, 0 to 2% by weight zinc, 0 to 1% by weight tin, 0 to 3% by weight indium and trace amounts of a grain refiner in combination with a veneer.

11. The dental bridge of claim 10, wherein said alloy is bonded to said veneer.

12. A dental crown formed of a dental alloy consisting essentially of 60 to 90% by weight gold, 0 to 20% by weight silver, 0 to 7% by weight palladium, 0 to 11% by weight platinum, 0 to 20% by weight copper, 0 to 2% by weight zinc, 0 to 1% by weight tin, 0 to 3% by weight indium and trace amounts of a grain refiner in combination with a veneer.

13. The dental crown of claim 12, wherein said alloy is bonded to said veneer.
